# EUROPEAN PATENT SPECIFICATION

(11) **EP 4 683 558 B1**
(45) Date of publication and mention of the grant of the patent: **04.11.2026**
(21) Application number: 24715239.0
(22) Date of filing: 19.03.2024
(51) Int. Cl.: A61B 5/024, A61B 5/026, A61B 5/0285, A61B 5/00

(54) **OPTICAL APPARATUS AND OPTICAL METHOD**
OPTISCHE VORRICHTUNG UND OPTISCHES VERFAHREN
APPAREIL OPTIQUE ET PROCÉDÉ OPTIQUE

(30) Priority: 20.03.2023 FI 20235320
(43) Date of publication of application: 28.01.2026
(73) Proprietor: Oulun yliopisto, 90570 Oulu (FI)
(72) Inventor: NISSINEN, Jan, 90570 Oulu (FI); HUIKARI, Jaakko, 90570 Oulu (FI); NISSINEN, Ilkka, 90570 Oulu (FI)
(74) Representative: Kolster Oy Ab
(86) International application number: PCT/FI2024/050126
(87) International publication number: WO 2024/194527

(56) References cited:
- US-A1- 2016 022 157
- US-A1- 2020 037 956
- US-A1- 2023 050 179

## Description

### Field

The invention relates to an optical apparatus and an optical method.

### Background

The blood pulse wave velocity (PWV) is based on at least two moments of detections of a pulse wave at at least two different points in the blood vessels and a measurement of the travelled distance of the pulse wave between the points. The measurement may use only two points and an estimation that the pulse wave has travelled a path of a straight line between the points. With these assumptions a speed of the blood wave velocity v is the distance travelled s divided by the difference in time t of detections: v=s/t.

A pressure sensor, such as a tonometer, is considered the gold standard for measuring PWV, but some operating skill is required to use it in order to have proper results. Traditionally, optical PPG device with one or more continuous wave (CW) LEDs and driver components and two or more photodetectors with the separate receiver channels have been used. However, the more components are used, the larger the size and the power consumption of the measurement device is. In addition, a pulse wave of blood distributes in the arterial tree to blood vessels of different sizes, which causes the speed of the pulse wave obtained to be unreliable. Namely, the speed of the pulse wave depends on the size of the blood vessel and receiving signals from pulse waves of a plurality of blood vessels of different sizes may lead to poor measurement results. Hence, an improvement in the measurement would be welcome. Optical blood flow measurement techniques are known from US 2023/050179 A1 and US 2016/022157 A1.

### Brief description

The present invention seeks to provide an improvement in the measurements.

The invention is defined by the independent claims. Embodiments are defined in the dependent claims.

### List of drawings

Example embodiments of the present invention are described below, by way of example only, with reference to the accompanying drawings, in which
Figure 1 illustrates an example of an application of the optical apparatus;
Figure 2 illustrates an example where fluid flowing in a fluid channel of a living body is measured;
Figure 3 illustrates an example of an array of single-photon avalanche detectors;
Figure 4 illustrates an example of numbers of photons detected by the two separate arrays;
Figure 5A illustrates an example of a histogram which shows a number of detected photons as a function of time windows;
Figure 5B illustrates an example of relation between time-of-flights of time windows of a histogram and depths within tissue;
Figure 5C illustrates an example of distributions of numbers of photons as a function of time relating to Fig. 4;
Figure 6 illustrates an example of a block chart of the optical apparatus;
Figure 7 illustrates an example of a data processing unit; and
Figure 8 illustrates of an example of a flow chart of a measuring method.

### Description of embodiments

The following embodiments are only examples. Although the specification may refer to "an" embodiment in several locations, this does not necessarily mean that each such reference is to the same embodiment(s), or that the feature only applies to a single embodiment.

Single features of different embodiments may also be combined to provide other embodiments. Furthermore, words "comprising" and "including" should be understood as not limiting the described embodiments to consist of only those features that have been mentioned and such embodiments may also contain features/structures that have not been specifically mentioned. All combinations of the embodiments are considered possible if their combination does not lead to structural or logical contradiction.

The term "about" means that quantities or any numeric values are not exact and typically need not be exact. The reason may be tolerance, resolution, measurement error, rounding off or the like, or a fact that the feature of the solution in this document only requires that the quantity or numeric value is approximately that large. A certain tolerance is always included in real life quantities and numeric values.

It should be noted that while Figures illustrate various embodiments, they are simplified diagrams that only show some structures and/or functional entities. The connections shown in the Figures may refer to logical or physical connections. It is apparent to a person skilled in the art that the described apparatus may also comprise other functions and structures than those described in Figures and text. It should be appreciated that details of some functions, structures, and the signalling used for measurement and/or controlling are irrelevant to the actual invention. Therefore, they need not be discussed in more detail here.

In this application, the term "determine" in its various grammatical forms may mean calculating, computing, data processing for deriving a result, looking up in a database or the like. As a result "determine" may also mean select, choose or the like.

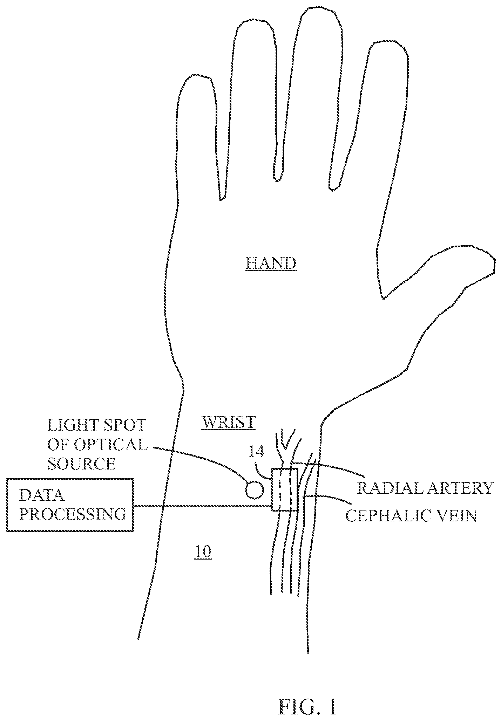

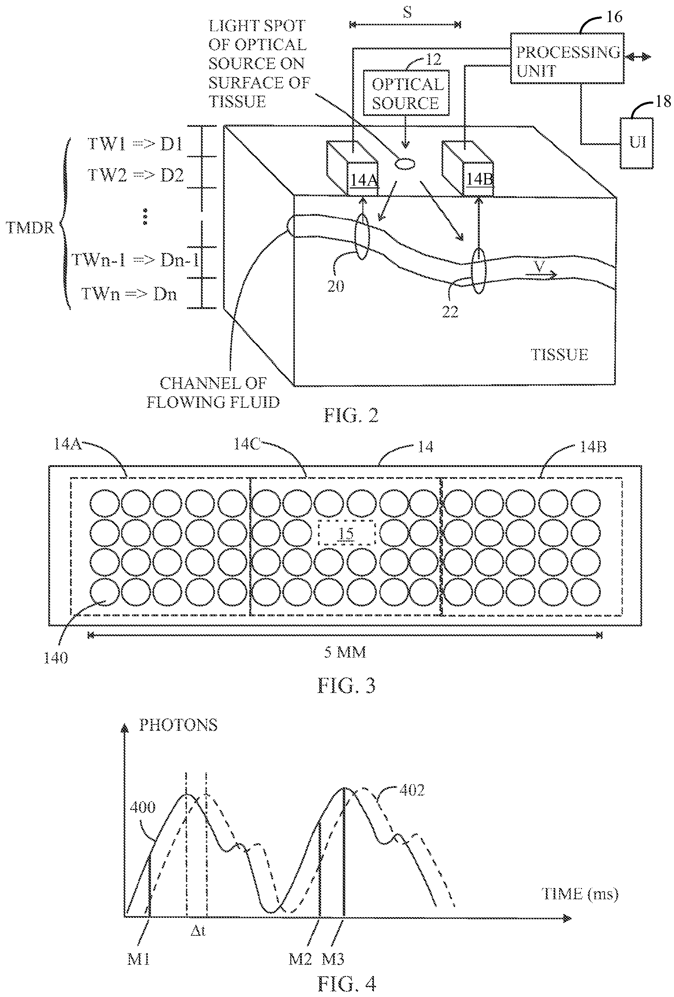

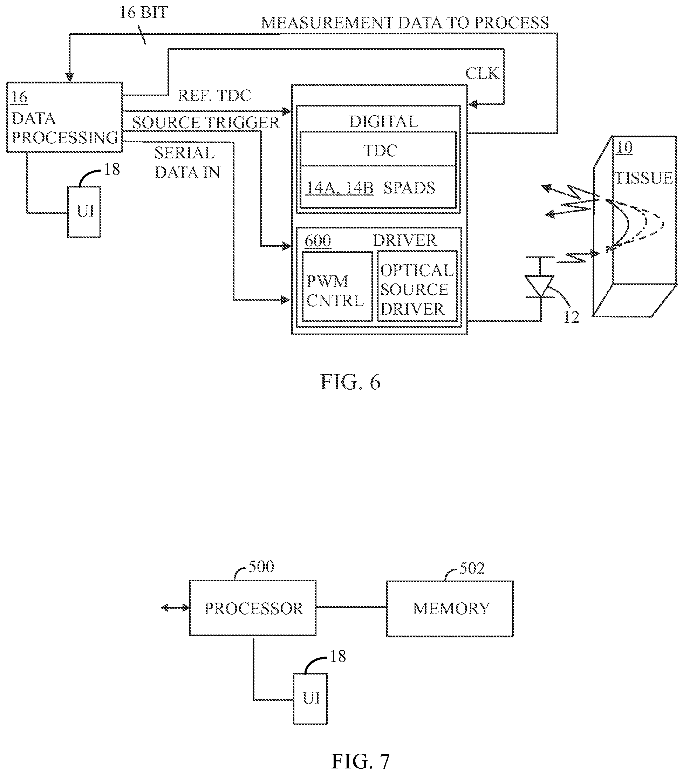

Examine now an optical apparatus based on Figs 1, 2, 3 and 6. Fig. 1 illustrates an example of a measurement of fluid flow within a living tissue 10, the example showing a measurement from a wrist. Fig. 2 illustrates an example where fluid such as blood flowing in a fluid channel such as a blood vessel of a living body is measured. Fig. 3 illustrates an example of an array of detectors. Fig. 6 illustrates a block diagram of the optical apparatus.

The optical apparatus for the fluid flow measurement comprises an optical radiation source 12 (not shown in Fig. 1, see Figs 2 and 6) that outputs an optical pulse into the tissue 10 at successive moments. The optical radiation source may comprise one or more light emitting diodes or lasers, for example. The optical radiation source 12 may output the optical pulses repeatedly in a regular or irregular manner. The optical radiation source 12 outputs optical pulses at an output rate F₁, where the rate F1 may be 1 MHz or more, for example, without limiting to this. The duration of a single optical pulse may be in a range 1 ns to 1000 nanoseconds or 1 ps to 1000 picoseconds or something between these, for example.

The optical apparatus also comprises a detector array 14. At least one first array 14A of single-photon avalanche detectors of the detector array 14 detects, for each of the successive moments of the outputs of the optical pulse, photons from an interaction between the optical pulse and the fluid flow at a first location 20 of the flow. Additionally, the optical apparatus comprises at least one second array 14B of single single-photon avalanche detectors that detect, for each of the successive moments of the successive outputs of the optical pulse, photons from interaction between the optical pulse and the fluid flow at second location 22 of the flow. The single-photon avalanche detectors operate in a Geiger-mode for detecting single photons. In the Geiger-mode, the semiconductor detector is biased above its reverse-bias breakdown voltage. A Geiger-mode detector is in that manner not a linear amplifier, which detection of single photons. A person skilled in the art is familiar with single-photon avalanche detectors and their Geiger-mode, *per se.*

The second location 22 is in an upstream or downstream location of the flow in the channel of flowing fluid within the tissue 10 with respect to the first location 20. This means that a distance travelled with the flow between a source or beginning of the flow and the first location may be shorter than a distance travelled with the flow between said source and the second location. Alternatively, a distance travelled with the flow between the source or beginning of the flow and the first location may be longer than a distance travelled with the flow between said source and the second location. The source of the flow can be considered the heart for a flow of blood. Inside bowels, for example, there is not necessarily a single source of the flow because of peristalsis in intestines, for example. Still, the mouth can be considered the beginning of the flow. That is, the channel through which the fluid flows has a beginning (and an end) and they can be used for references in determination of the separate locations of the different arrays.

In an embodiment an example of which is illustrated in Fig. 3, the first and second array 14A, 14B may be sections of a single array 14. The light of the optical radiation source 12 may go into the tissue 10 beside the array 14 as shown in Figs. 1 and 2 or there may be an opening 15 in the array 14 for allowing the light from the optical radiation source 12 to propagate through the opening 15 into the tissue 10. The wavelength band that is detected should be sensitive to a variation of a property of the fluid. The property may absorption of the fluid. The property may then relate to pressure, density, and/or consistency of the fluid. Blood as an example of fluid flows in a pulsated manner. As a result, the flow has pulse waves which are regular variations of the blood. The variation, however, does not need to be regular. It is enough for the measurement of speed of the flow that a same variation is detected at different moments by the first and second array 14A, 14B of known geometry. In an embodiment, sized of pixels of the first and second array 14A, 14B may be known. Note that the pixels may have the same sizes or different sizes. In an embodiment, distances of the pixels may be known, and also in this case the distances may be equal or different. In an embodiment, a spatial distance between the at least one first and second array (14A, 14B) may be known. For a measurement of a rhythm of the variation of the fluid, such as related to the heartbeat, for example, it is enough that at least one of the first and the second array 14A, 14B detect the variation. A person skilled in the art is familiar with a suitable sensitivity related to the fluid and can choose the optical source 12 and the first and second arrays 14A and 14B suitably. The detected wavelength band may be optically band-pass filtered in order to make a desired optical band to propagate from the tissue 10 to the first and second array 14A, 14B. The band-pass filtering may make the optical band that is detected narrower than the optical band that is output by the optical source 12.

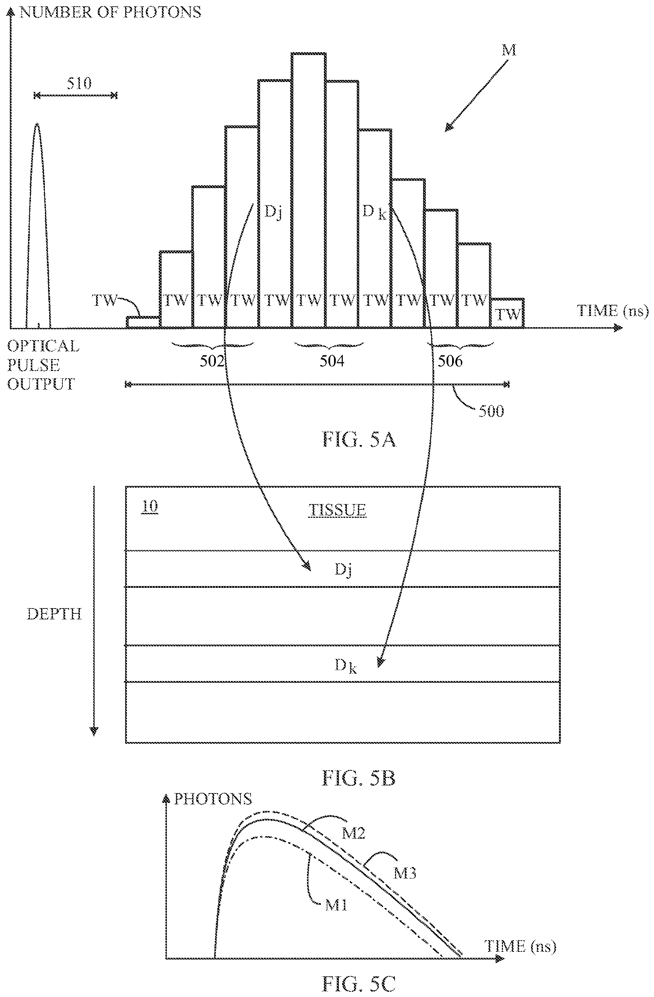

The optical apparatus further comprises a data processing circuitry 16 that counts photons based on their detections by the detector array 14. A sampling rate for detecting the photons of the optical pulses may be F₂. In an embodiment, the sampling rate may be about 100 Hz to about 10000 Hz on average, for example. In an embodiment, the sampling rate for detecting the photons of the optical pulses may be in a range about 500 Hz to 1000 Hz on average, for example. As an example, the optical radiation source 12 may output one million optical pulses per second, which is the output rate F₁ of the optical pulses. Then the sampling rate F₂ of the data processing circuitry 16 for counting the photons for each measurement may be one kilohertz, the counting for each measurement lasting for a millisecond, for example. In this manner, each measurement may comprise photons from F₁/F₂ optical pulses, for example, which is one thousand optical pulses in this example. As a result, the measurement accumulates photons of a plurality of detections, and in each of the detections by the detector array 14, the photons may come from different depths of the tissue. The accumulation may be converted in an average over a unit of time, for example. The deeper from the tissue the photons come, the longer is their time-of-flight (see Figs 5A to 5C).

In an embodiment, one measurement may include an accumulation of detections of photons of 10000 optical pulses or any desired number of optical pulses. In an embodiment, the measurements may be performed in a burst mode, where a burst has a desired number of optical pulses, and between two directly adjacent bursts there is a period or delay where no optical pulses are transmitted. More than one measurement may be averaged for a final result.

The data processing circuitry 16 that determines a speed of the flow based on a known spatial distance between the at least one first and second array 14A, 14B, and a similarity between numbers of photons detected by the at least one first and second array 14A, 14B at a plurality of moments of outputs of the optical pulse. The similarity may be measured between the numbers of photons detected by the at least one first array 14A and the at least one second array 14B. The similarity of numbers of detected photons may refer to the numbers of photons detected within a period dedicated for detections that is a measurement period 500 (see Fig. 5A). In this manner, the similarity is measured based on numerical values of a total number of detected photons. Alternatively or additionally, the similarity of numbers of photons may refer to a temporal distribution of the numbers of photons detected within the measurement period 500 for detections.

The single-photon avalanche detectors may perform a plurality of temporally successive measurements in time windows TW for a single optical pulse and repeat the plurality of measurements for two or more temporally successive optical pulse outputs by the optical source 10. This kind of measurement allows comparison of numbers of detected photons as a function of time, and it may be considered this measurement performs the similarity determination based on a shape of the distribution of time-of-flight of detected photons, the shape depending on the number of detected photons at each moment of measurement or at each time window TW for a single optical pulse. As a result, it can be defined that the shape depends on the number of detected photons at each bin of photons' time histogram. Shape comes from numbers of detections/photons at each moment. Even a number of photons at a single moment can be thought to be a shape. The shape can also be an envelope of the peak numbers of photons.

Fig. 4 illustrates an example of numbers 400 of photons detected by the first array 14A and numbers 402 of photons detected by the second array 14B as a function of time. The y-axis represents numbers of detected photons and the x-axis represents time in arbitrary scales. Note that durations of measurements examples of which are M1, M2 and M3, may be hundreds of nanoseconds to a few milliseconds, for example (compare time scale with those of Figs 5A and 5C). Fig. 4 illustrates the numbers of photons as continuous curves but in reality curves may be measured like illustrated in Fig. 5A in a discrete form. The measurement of the flow with a plurality of optical pulses should continue long enough for a property of the flow such as the pulse wave to travel fully a spatial distance S between the first and second array 14A, 14B. It can be noticed in Fig. 4 that a continuing measurement produces two curves that resemble each other but they have a high similarity with a phase shift that corresponds to a delay *Δt.* The data processing circuitry 16 searches for a maximum similarity of numbers of photons and defines the delay Δ*t* related to it based on timings of detections of the photons. The delay Δ*t* is the time that it takes for the flow to travel the distance between the first and the second array 14A, 14B. When a plurality of measurements is averaged, the total time for the measurements may be the longer the more averaging is wished or required for.

In an embodiment, the at least one first array 14A and the at least one second array 14B may be movable with respect to the tissue 10. Simultaneously with movement of the arrays 14A, 14B on the tissue 10, the data processing circuitry 16 may count the number of photons detected by the at least one first array 14A and the number of photons detected by the at least one second array 14B) synchronously with each of the outputs of the optical pulse. That means, when an optical pulse is output into the tissue 10, the photons coming to the first array 14A and the second array 14B are counted. A total number of photons may be counted in the whole measurement period 500, or a number of photons in different time windows TW within the measurement period 500 may be counted. The latter may result in a histogram which shows a number of detected photons as a function of time as illustrated in Fig. 5A. After that a next optical pulse is output and the number of photons of this optical pulse coming to the first array 14A and the second array 14B are counted. That kind of counting of the photons optical pulse per optical pulse is performed continuously. The numbers of detected photons relating to one or more corresponding time windows TW of two or more measurements differing by *Δt* on average will resemble each other. Time Δt is the time that it takes for the detectable property of the fluid to travel with the flow the distance Δ*s* relating the spacing of the first array 14A and the second array 14B. As a result, the data processing circuitry 16 can estimate the velocity v of the flow to be about *v=*Δ*s*/Δ*t.* If a direction of the flow of the fluid is known to have an angle with respect to a longitudinal direction between the first and the second arrays 14A, 14B, it can be taken into account by applying a suitable mathematical means such as trigonometry and potentially also integration.

When the first and second arrays 14A, 14B are moved on the tissue 10, the data processing circuitry 16 may form information on the numbers of photons detected by the arrays 14A, 14B as a function of a location relating to the tissue 10 for guiding a user to place the arrays 14A, 14B at locations where the flow is detectable for the measurement of the speed of the flow. The data processing circuitry 16 may also present the information with a user interface 18. The user of the optical apparatus in general may be a human being. Alternatively or additionally, the user may be a machine of artificial intelligence and it may potentially apply machine learning for starting and potentially improving its operation in at least one of analyzing the measurements and guiding the mechanical movements of the first and second array 14A, 14B. In an embodiment, the optical apparatus includes the machine of artificial intelligence and/or machine learning. In an embodiment, the machine may comprise mechanical movement means such as at least one kinetic energy source which may be a motor or the like, and transmission mechanics for moving the first and the second arrays 14A, 14B on the living tissue with the kinetic energy. A person skilled in the art is familiar with the kinetic energy source and the transmission mechanics, *per se.*

The speed of the pulse wave of the blood may depend on the size of the blood vessel, and receiving signals from pulse waves of a plurality of blood vessels of different sizes may lead to a different result than measuring the speed of the pulse wave in a single blood vessel or in a reduced number of blood vessels. Including improvement in depth resolution and/or a measurement at locations where the flow clearly detectable enables a reliable measurement. Additionally, signals from different depths can be separated. Based on this, also blood pressure can be measure accurately.

The animal or human tissue has a variety of channels through which various fluids flow. The channels themselves are not necessarily of much interest although their depth in the tissue 10 may need to be determined. In an embodiment, the fluid may be: blood, contrast medium, interstitial fluid of a plant, animal or a human being, lymphatic fluid, fluid of metabolism, seminal fluid, liquor amnii, gas, mass of an alimentary tract or liquid of a urinary tract. Sometimes it may be possible or desirable that the fluid is some combination of these. The gas may be within a windpipe or elsewhere within the body.

The tissue 10 of a plant typically comprise dermal and ground layers. The dermal tissue, which may also be called epidermis, is on the surface of the plant. The tissue includes water-transporting xylem and food-transporting phloem and they are channels for a flow of fluid in the plant.

Fig. 5A illustrates an example of numbers of detected photons in a plurality of time windows TW, the detections of photon being done by the first array 14A, for example. A similar kind of distribution of photons can be formed based on detections of photons by the second arran 14B. The number of photons are in the y-axis and time is in the x-axis. Both axes are in arbitrary scales. However, note that a width of the time windows TW may be a few nano seconds, for example, based on a time of flights of the photons from the tissue 10. The time scales of Fig. 4 and Figs. 5A (and 5C) thus have a ratio F1/F2 because a plurality of optical pulses are needed for each of the measurements examples of which are M1, M2 and M3.

In an embodiment of Fig. 5A, the data processing circuitry 16 may count the number of photons at one or more time windows TW within a measurement period 500 in conjunction with each of the repeated moments of outputs of the optical pulse. The data processing circuitry 16 may then form information on a number of photons in one or more depths within the tissue 10 as additionally illustrated in Fig. 5B, because each one of the time windows TW within the measurement period 500 corresponds to single depth range D*ᵢ* of a total measured depth range TMDR within the tissue 10, where *i* is an index from 1 to *n* (see also Fig. 2). In Figs 5A and 5B, depths D*ⱼ* and D*ₖ* are shown as examples, where *j* and *k* are indices of depths.

In an embodiment, more than one depth range D*ₖ* to D*ⱼ* may be measured, where *k* and *j* are indices between 1 and *n*, and such a combination of depth ranges may be called a measurement section in the temporal dimension. There may be one measurement section 502, 504, 506 that is measured or more than one measurement sections 502, 504, 506 that are measured in response to each single optical pulse of the successive optical pulses. In such a case, the measurement is performed over several time windows TW. Information received from the measurement may be used for guiding the user to measure the speed of a flow within one or more desired depth ranges where the fluid of interest flows. Namely, the flow of the fluid may take place at a certain depth and the measurement performed at that depth has a stronger signal related to the flow than a measurement at another depth(s). The stronger signal refers to a number of photons relating to the flow, and a variation of the number of photons is higher than in other depths which also typically leads to a higher similarity than the similarity measured from other depths where no flow or some other flow of fluid is present. The flow of interest can be detected based on a regular or expectedly determined variation of the number of photons (see Fig. 4). It may be assumed that the tissue 10 causes random variation in the number of photons and its amplitude is lower than that caused by the detectable property such as pulse waves of the flow of the fluid.

Fig. 5C illustrates an example of distributions of numbers of photons as a function of time relating to Fig. 4. A measurement M1 detects the least photons of these three example measurements. That is why its level is lowest in both Figs 4 and 5C. A measurement M2 detects a middle number of photons of these three example measurements. That is why its level is between the two other measurements of these three example measurements in both Figs 4 and 5C. A measurement M3 has the highest number of detected photons of these three example measurements and that causes it to be at the highest level in both Figs 4 and 5C. This example illustrates a possibility that all detected photons of the measurement period 500 are utilized for obtaining the variation of the detected photons. However, a similar variation may be achieved even more efficiently by utilizing detections of numbers photons of a certain depth range such as one or more measurement sections 502, 504, 506 where the flow of interest is assumed, estimated or known to be.

In an embodiment, the data processing circuitry 16 may count the number of photons detected by the at least one first array 14A and the at least one second array 14B at one or more time windows TW within a measurement period 500 in conjunction with each of the successive moments of output of the optical pulse. The data processing circuitry 16 may then determine and set at least one time window TW within the measurement period 500 for the measurement of the speed of the flow at each depth range D*ₖ* corresponding to said at least one time window TW. This allows the measurement of the flow of fluid at a depth where the flow of the fluid occurs. In this manner, disturbance of the measurement from other parts of the tissue 10 can be eliminated or alleviated.

In an embodiment, the data processing circuitry 16 may determine, for each of the successive moments in conjunction with each of the outputs of the optical pulse, a time interval 510 between an output of the optical pulse and a reception of photons of the optical pulse interacted with the flow separately for the at least one first array 14A and the at least one second array 14B. The longer the time interval 510 is, the later the data processing circuitry 16 starts counting the photons. That also means that the longer the time interval 510 is, the deeper the first photons of the measurement come from the tissue 10. In that manner, it is possible to adjust the measurement depth to the level of the flow of the fluid of interest. In a similar manner, the data processing circuitry 16 may determine a length of the measurement period 500. By adjusting the time interval 510 and the measurement period 500, one or more time windows TW at any depth in the tissue 10 can be selected for the measurement. This is one way to select one or more measurement sections 502, 504, 506.

In an embodiment, the data processing circuitry 16 may determine the speed of the flow based on the similarity between temporal distributions of numbers of detections of photons by the at least one first array 14A and the at least one second array 14B at a plurality of successive moments within a measurement period 500 in conjunction with at least one of the successive moments when optical pulses are output by the optical source 12. The delay Δt between the measurements is determined based on the maximum similarity. If the maximum similarity is at or lower than about 0.7 in the scale 0 to 1, for example, the data processing circuitry 16 may determine the measurement unreliable. However, the numerical value is merely an example and some other value may also be use.

In an embodiment an example of which is illustrated in Fig. 3, the at least one first array 14A and the at least one second array 14B may be integrated together as a single component of an array 14 of solid material. The array 14 may have single-photon avalanche detectors 140 in the whole length but the single-photon avalanche detectors in a middle section 14C may be out of use (only one single-photon avalanche detector has reference number 140, but all circles refer to the single-photon avalanche detectors in Fig. 3). The single-photon avalanche detectors of the first and second array 14A and 14B may be on both sides of the single-photon avalanche detectors of the middle section 14C.

In an embodiment an example of which is illustrated in Fig. 6, an electrical driver 600 of the optical radiation source 10, the first and second array 14A, 14B and at least a part of the data processing circuitry 16 may be fabricated on semiconducting material of a single die. The part of the data processing circuitry 16 may be a time-to-digital converter (TDC), for example. As shown in Fig. 6, the data processing circuitry provides timing for the detection and operation. The time-to-digital converter receives clock (CLK) and reference to TDC, which keeps the timing of the TDC stable in variable conditions, for example, and optical source triggering outputting optical pulses. The electrical driver 600 of the optical source 12 may also receive a pulse width control from the data processing circuitry 16. The time-to-digital converter TDC provides the data processing circuitry 16 with information on timings of the detections of photons scattered from the tissue to the first and the second array 14A, 14B.

In an embodiment, optical radiation source 12 may output repeatedly optical pulses of near-infrared light. For example, about 750 nm, about 800nm and/or about 860 nm may be used because those wavelengths can penetrate much deeper in a human and animal tissue 10 than visible light. For plants the wavelengths may also work. Blood volume is changing when heart is pumping more blood in arteries and this is changing the number of photons that are detected by the first and second array 14A, 14B. In this way, beating of heart may also be revealed from a temporal variation of detected photons.

In an embodiment, the fluid is blood and the data processing circuitry 16 may detect a heartbeat and determine a heart rate based on a characteristic variation of the numbers of photons with the heartbeat as a function of time. Fig. 4 illustrates the characteristic variation of the detected photons when flow of blood is measured, and the variation is caused by the heartbeat. An inverse of time between two peaks of signals from the same array 14A or 14B represents a frequency of the heartbeat. A plurality of inverses of periods between peaks may be averaged in order to have a mean heart rate.

In an embodiment, optical radiation source 12 may output repeatedly the optical pulse of near-infrared light in one or more bands that are chosen based on at least one of the following: optical properties of the tissue and optical properties of the fluid.

In an embodiment, the data processing circuitry 16 may determine the speed of the flow as a ratio between the known distance and a temporal difference between detections of photons made by the at least one first array 14A and the at least one second array 14B, the temporal difference being based a peak of similarity.

In an embodiment an example of which is illustrated in Fig. 7, the data processing unit 16 of the optical apparatus comprises one or more processors 700, and one or more memories 702 including computer program code. The data processing unit 16 can be considered a computer. The one or more memories 702 and the computer program code cause, with the one or more processors 700, the optical apparatus at least to determine the speed of the flow based on the known spatial distance S between the at least one first and second array 14A, 14B, and the similarity between numbers of photons detected by the at least one first and second array 14A, 14B at the plurality of moments of outputs of the optical pulse.

The term "computer" includes a computational device that performs logical and arithmetic operations. For example, a "computer" may comprise an electronic computational device, such as an integrated circuit, a microprocessor, a mobile computing device, a laptop computer, a tablet computer, a personal computer, or a mainframe computer. A "computer" may comprise a central processing unit, an ALU (arithmetic logic unit), a memory unit, and a control unit that controls actions of other components of the computer so that steps of a computer program are executed in a desired sequence. A "computer" may also include at least one peripheral unit that may include an auxiliary memory (such as a disk drive or flash memory), and/or may include data processing circuitry.

A user interface 18 means an input/output device and/or unit. Nonlimiting examples of a user interface include a touch screen, other electronic display screen, keyboard, mouse, microphone, handheld electronic controller, digital stylus, display screen, speaker, and/or projector for projecting a visual display.

What is explained before this refers to time resolved diffuse optics where a short optical pulse is transmitted to tissue 10 and backscattered photons are received by the single-photon avalanche diode array 14. Timing electronics such as TDC measures the time-of-flight of the photons. For example, blood volume varying in response to heartbeat. This variation can be detected as a variation of backscattered photons. This allows the measurement of flow of the blood and additionally also the heart rate. It is possible to fabricate the laser diode driver, the SPAD array and the timing electronics in a single complementary metal-oxide semiconductor (CMOS) die. In an embodiment, a blood pulse is measured by the first and second SPAD array 14A, 14B measures the blood pulse at the wrist while the first and the second SPAD arrays 14A, 14B have at the small physical distance therebetween. The physical distance may be about 4mm to about 8 mm, for example. In an embodiment, the number of TDCs may be the same as the number of the SPADs. In that manner, one optical pulse may cause a plurality of measured time-of-flights.

In an embodiment, the optical source driver may drive two laser diodes outputting different wavelengths separately.

It is possible to move the SPAD arrays 14A, 14B on the tissue 10 and to find a desired artery, for example. Because of a possibly fast repetition rate of the optical source 12, a high sampling rate can be achieved which allows the pulse wave velocity to be measured locally using a short distance between the SPAD arrays 14A, 14B. Moreover, the delay Δ*t* of the blood pulse between these two points is caused by the blood pulse wave velocity v and also depending on the distance S of these two separate SPAD arrays 14A, 14B. Because the time-resolved technology allows to find a desired artery or the like from the wrist, for example, an accurate pulse wave velocity v can be determined. Note also that, a continuous illumination of a photoplethysmogram (PPG) of the prior art gives an average pulse wave velocity of blood and cannot separate if the signal comes from an artery or a venous, so that the waves obtained by this method do not result in a reliable information on what pulse wave velocity is actually measured.

Assume now that a sampling frequency is about 1000Hz, which means a temporal distance of the samples is about 1ms, at least on an average. Additionally assume that a spatial distance between two detectors is about 10 mm. Then if a delay about 10ms results in a highest similarity of the number of detections of photons and/or the temporal distributions of numbers of detections of photons, a speed of flow v is about v=0,01m/10ms=1m/s. With a plurality of measurements, the delay can be an average such that the delay of 10 ms on average results in a highest similarity of the number of detections of photons and/or the temporal distributions of numbers of detections of photons.

A similarity measurement measures if data of the first array 14A is similar to data of the second array 14B. Similarity can be considered to measure if two or more data sets are related or if they are unrelated. The similarity may refer to a number of measured detections of photons or similarity of the temporal distribution of the numbers of photons. The temporal distributions of the numbers of photons may be in the form of time series of each histogram bin. The similarity may be measured based on correlation and Euclidian distance, for example. The similarity may be expressed using a value of a numerical system. The value may be expressed in percentages, for example. The numerical value typically increases when the compared data resemble more and more each other. A person skilled in the art is familiar with similarity measurements, *per se.*

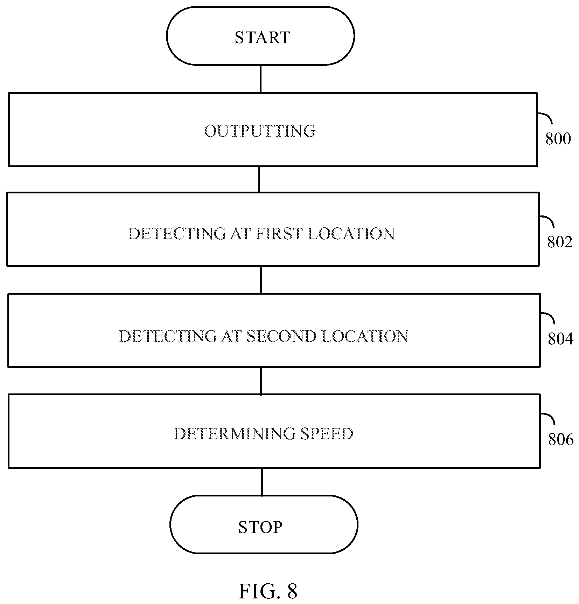

Figure 8 is a flow chart of the optical method of measuring fluid flow within a living tissue 10. In step 800, an optical pulse is output into the tissue 10 at successive moments by an optical radiation source 12.

In step 802, photons from an interaction between the optical pulse and the fluid flow at a first location of the flow are detected for each of the successive moments of the outputs of the optical pulse by at least one first array 14A of single-photon avalanche detectors.

In step 804, photons from interaction between the optical pulse and the fluid flow at a second location of the flow are detected for each of the successive moments of the successive outputs of the optical pulse by at least one second array 14B of single single-photon avalanche detectors, the second location being in an upstream or downstream location with respect to the first location.

In step 806, a speed of the flow is determined based on a known spatial distance between the at least one first and second array 14A, 14B, and a similarity between numbers of photons detected by the at least one first and second array 14A, 14B at a plurality of moments of outputs of the optical pulse by a data processing circuitry 16.

The method shown in Figure 8 may be implemented, at least partly, as a logic circuit solution or computer program. The computer program may be placed on a computer program distribution means for the distribution thereof. The computer program distribution means is readable by a data processing device, and it encodes the computer program commands, carries out the measurements and optionally controls the processes on the basis of the measurements.

The computer program may be distributed using a distribution medium which may be any medium readable by the controller. The medium may be a program storage medium, a memory, a software distribution package, or a compressed software package. In some cases, the distribution may be performed using at least one of the following: a near field communication signal, a short distance signal, and a telecommunications signal.

It will be obvious to a person skilled in the art that, as technology advances, the inventive concept can be implemented in various ways. The invention and its embodiments are not limited to the example embodiments described above but may vary within the scope of the claims.

## Claims

1. An optical apparatus for measuring fluid flow within a living tissue (10), wherein the apparatus comprises
an optical radiation source (12) configured to output an optical pulse into the tissue (10) at successive moments;
at least one first array (14A) of single-photon avalanche detectors configured to detect, for each of the successive moments of the outputs of the optical pulse, photons from an interaction between the optical pulse and the fluid flow at a first location of the flow;
at least one second array (14B) of single single-photon avalanche detectors configured to detect, for each of the successive moments of the successive outputs of the optical pulse, photons from interaction between the optical pulse and the fluid flow at second location of the flow, the second location being in an upstream or downstream location with respect to the first location; and
a data processing circuitry (16) configured to determine a speed of the flow based on a known geometry of the at least one first array (14A) and the at least one second array (14B), and a similarity between numbers of photons detected by the at least one first and second array (14A, 14B) at a plurality of moments of outputs of the optical pulse.

2. The apparatus of claim 1, **characterized in that** the at least one first array (14A) and the at least one second array (14B) are movable with respect to the tissue (10), and simultaneously with movement of the arrays (14A, 14B) the data processing circuitry (16) is configured to count a number of photons detected by the at least one first array (14A) and a number of photons detected by the at least one second array (14B) synchronously with each of the outputs of the optical pulse; and
the data processing circuitry (16) is configured to form and present information on the numbers of photons detected by the arrays (14A, 14B) as a function of a location relating to the tissue (10) for guiding a user to place the arrays (14A, 14B) at positions where the flow is detectable for the measurement of the speed.

3. The apparatus of claim 1, **characterized in that** the data processing circuitry (16) is configured to count a number of photons detected by the at least one first array (14A) and the at least one second array (14B) at one or more time windows (TW) within a measurement period (500) in conjunction with each of the successive moments of output of the optical pulse, and form and present information on a number of photons in one or more depths within the tissue (10), each one of the time windows (TW) within the measurement period (500) corresponding to single depth range (D*i*) of the total measurement depth range (TMDR) within the tissue (10) for guiding the user to measure the speed of a flow within one or more desired depth ranges (D*k* to D*j*).

4. The apparatus of claim 1, **characterized in that** the data processing circuitry (16) is configured to count a number of photons detected by the at least one first array (14A) and the at least one second array (14B) at one or more time windows (TW) within a measurement period (500) in conjunction with each of the successive moments of output of the optical pulse, and determine at least one time window (TW) within the measurement period (500) for measuring the speed of the flow at each depth range (D*i*) corresponding to said at least one time window (TW).

5. The apparatus of claim 1, **characterized in that** a data processing circuitry (16) is configured to determine, for each of the successive moments in conjunction with each of the outputs of the optical pulse, a time interval (510) between an output of the optical pulse and a reception of photons of the optical pulse interacted with the flow separately for the at least one first array (14A) and the at least one second array (14B).

6. The apparatus of claim 1 or 5, **characterized in that** the data processing circuitry (16) is configured to determine the speed of the flow based on the similarity between temporal distributions of numbers of detections of photons by the at least one first array (14A) and the at least one second array (14B) at a plurality of successive moments within a measurement period in conjunction with at least one of the successive moments of output of the optical pulse.

7. The apparatus of claim 1, **characterized in that** the at least one first array (14A) and the at least one second array (14B) are integrated together as a single array (14) of solid material.

8. The apparatus of any of the preceding claims, **characterized in that** an electrical driver (600) of the optical radiation source (10), the first and second array (14A, 14B) and at least a part of the data processing circuitry (16) are fabricated on semiconducting material of a single die.

9. The apparatus of any of the preceding claims, **characterized in that** the fluid is at least one of the following: blood, contrast medium, interstitial fluid, lymphatic fluid, fluid of metabolism, seminal fluid, liquor amnii, gas, mass of an alimentary tract and liquid of a urinary tract.

10. The apparatus of any of the preceding claims, **characterized in that** the optical radiation source (12) configured to output repeatedly the optical pulse of near-infrared light.

11. The apparatus of any of the preceding claims, **characterized in that** optical radiation source (12) configured to output repeatedly the optical pulse of near-infrared light in one or more bands that are chosen based on at least one of the following: optical properties of the tissue and optical properties of the fluid.

12. The apparatus of any of the preceding claims, **characterized in that** the data processing circuitry (16) is configured to determine the speed of the flow as a ratio between the known distance and a temporal difference between detections of photons made by the at least one first array (14A) and the at least one second array (14B), the temporal difference being based on a peak of similarity.

13. The apparatus of any of the preceding claims, **characterized in that** the fluid is blood and the data processing circuitry (16) is configured to determine a heartbeat based on a characteristic variation of the numbers of photons as a function of time.

14. The apparatus of any of the preceding claims, **characterized in that** the data processing circuitry (16) comprises one or more processors (500), and one or more memories (502) including computer program code; the one or more memories (502) and the computer program code configured to, with the one or more processors (500), cause apparatus at least to:
determine the speed of the flow based on the geometry of the at least one first and second array (14A, 14B), and the similarity between numbers of photons detected by the at least one first and second array (14A, 14B) at the plurality of moments of outputs of the optical pulse.

15. An optical method of measuring fluid flow within a living tissue, comprising
outputting (800), by an optical radiation source (12), an optical pulse into the tissue (10) at successive moments;
detecting (802), by at least one first array (14A) of single-photon avalanche detectors, for each of the successive moments of the outputs of the optical pulse, photons from an interaction between the optical pulse and the fluid flow at a first location of the flow;
detecting (804), by at least one second array (14B) of single single-photon avalanche detectors, for each of the successive moments of the successive outputs of the optical pulse, photons from interaction between the optical pulse and the fluid flow at a second location of the flow, the second location being in an upstream or downstream location with respect to the first location; and
determining (806), by a data processing circuitry (16), a speed of the flow based on a known geometry of the at least one first and second array (14A, 14B), and a similarity between numbers of photons detected by the at least one first and second array (14A, 14B) at a plurality of moments of outputs of the optical pulse.

## Patentansprüche

1. Optische Einrichtung zum Messen einer Fluidströmung innerhalb eines lebenden Gewebes (10), wobei die Einrichtung Folgendes umfasst
eine optische Strahlungsquelle (12), die dazu ausgebildet ist, zu aufeinanderfolgenden Zeitpunkten einen optischen Impuls in das Gewebe (10) auszugeben;
mindestens ein erstes Array (14A) von Einzelphotonen-Lawinendetektoren, das dazu ausgebildet ist, für jeden der aufeinanderfolgenden Zeitpunkte der Ausgaben des optischen Impulses Photonen aus einer Wechselwirkung zwischen dem optischen Impuls und der Fluidströmung an einem ersten Ort der Strömung zu detektieren;
mindestens ein zweites Array (14B) von Einzelphotonen-Lawinendetektoren, das dazu ausgebildet ist, für jeden der aufeinanderfolgenden Zeitpunkte der aufeinanderfolgenden Ausgaben des optischen Impulses Photonen aus einer Wechselwirkung zwischen dem optischen Impuls und der Fluidströmung an einem zweiten Ort der Strömung zu detektieren, wobei der zweite Ort in Bezug auf den ersten Ort an einem stromaufwärts oder stromabwärts gelegenen Ort liegt; und
eine Datenverarbeitungsschaltung (16), die dazu ausgebildet ist, eine Geschwindigkeit der Strömung, basierend auf einer bekannten Geometrie des mindestens einen ersten Arrays (14A) und des mindestens einen zweiten Arrays (14B) und einer Ähnlichkeit zwischen Anzahlen von Photonen, die durch das mindestens eine erste und zweite Array (14A, 14B) an einer Vielzahl von Zeitpunkten von Ausgaben des optischen Impulses detektiert werden, zu bestimmen.

2. Einrichtung nach Anspruch 1, **dadurch gekennzeichnet, dass** das mindestens eine erste Array (14A) und das mindestens eine zweite Array (14B) relativ zu dem Gewebe (10) beweglich sind, und gleichzeitig mit einer Bewegung der Arrays (14A, 14B) die Datenverarbeitungsschaltung (16) dazu ausgebildet ist, synchron mit jeder der Ausgaben des optischen Impulses eine Anzahl von Photonen, die durch das mindestens eine erste Array (14A) detektiert werden, und eine Anzahl von Photonen, die durch das mindestens eine zweite Array (14B) detektiert werden, zu zählen; und
die Datenverarbeitungsschaltung (16) dazu ausgebildet ist, Informationen über die Anzahlen von Photonen, die durch die Arrays (14A, 14B) detektiert werden, als eine Funktion eines Ortes in Bezug auf das Gewebe (10) zu bilden und darzustellen, um einen Benutzer dahin zu führen, die Arrays (14A, 14B) an Positionen anzuordnen, an denen die Strömung für die Messung der Geschwindigkeit detektierbar ist.

3. Einrichtung nach Anspruch 1, **dadurch gekennzeichnet, dass** die Datenverarbeitungsschaltung (16) dazu ausgebildet ist, eine Anzahl von Photonen, die durch das mindestens eine erste Array (14A) und das mindestens eine zweite Array (14B) in einem oder mehreren Zeitfenstern (TW) innerhalb eines Messzeitraums (500) in Verbindung mit jedem der aufeinanderfolgenden Zeitpunkte der Ausgabe des optischen Impulses detektiert werden, zu zählen und Informationen über eine Anzahl von Photonen in einer oder mehreren Tiefen innerhalb des Gewebes (10) zu bilden und darzustellen, wobei jedes der Zeitfenster (TW) innerhalb des Messzeitraums (500) einem einzelnen Tiefenbereich (D*i*) des Gesamtmesstiefenbereichs (TMDR) innerhalb des Gewebes (10) entspricht, um den Benutzer dahin zu führen, die Geschwindigkeit einer Strömung innerhalb eines oder mehrerer gewünschter Tiefenbereiche (D*k* bis D*j*) zu messen.

4. Einrichtung nach Anspruch 1, **dadurch gekennzeichnet, dass** die Datenverarbeitungsschaltung (16) dazu ausgebildet ist, eine Anzahl von Photonen, die durch das mindestens eine erste Array (14A) und das mindestens eine zweite Array (14B) in einem oder mehreren Zeitfenstern (TW) innerhalb eines Messzeitraums (500) in Verbindung mit jedem der aufeinanderfolgenden Zeitpunkte der Ausgabe des optischen Impulses detektiert werden, zu zählen und mindestens ein Zeitfenster (TW) innerhalb eines Messzeitraums (500) zum Messen der Geschwindigkeit der Strömung bei jedem dem mindestens einen Zeitfenster (TW) entsprechenden Tiefenbereich (D*i*) zu bestimmen.

5. Einrichtung nach Anspruch 1, **dadurch gekennzeichnet, dass** eine Datenverarbeitungsschaltung (16) dazu ausgebildet ist, für jeden der aufeinanderfolgenden Zeitpunkte in Verbindung mit jeder der Ausgaben des optischen Impulses ein Zeitintervall (510) zwischen einer Ausgabe des optischen Impulses und einem Empfang von Photonen des optischen Impulses, die mit der Strömung wechselgewirkt haben, getrennt für das mindestens eine erste Array (14A) und das mindestens eine zweite Array (14B) zu bestimmen.

6. Einrichtung nach Anspruch 1 oder 5, **dadurch gekennzeichnet, dass** die Datenverarbeitungsschaltung (16) dazu ausgebildet ist, die Geschwindigkeit der Strömung, basierend auf der Ähnlichkeit zwischen zeitlichen Verteilungen von Anzahlen von Detektionen von Photonen durch das mindestens eine erste Array (14A) und durch das mindestens eine zweite Array (14B) an einer Vielzahl von aufeinanderfolgenden Zeitpunkten innerhalb eines Messzeitraums in Verbindung mit mindestens einem der aufeinanderfolgenden Zeitpunkte der Ausgabe des optischen Impulses zu bestimmen.

7. Einrichtung nach Anspruch 1, **dadurch gekennzeichnet, dass** das mindestens eine erste Array (14A) und das mindestens eine zweite Array (14B) zusammen als ein einzelnes Array (14) aus festem Material integriert sind.

8. Einrichtung nach einem der vorstehenden Ansprüche, **dadurch gekennzeichnet, dass** ein elektrischer Treiber (600) der optischen Strahlungsquelle (10), das erste und das zweite Array (14A, 14B) und mindestens ein Teil der Datenverarbeitungsschaltung (16) auf Halbleitermaterial eines einzelnen Dies hergestellt sind.

9. Einrichtung nach einem der vorstehenden Ansprüche, **dadurch gekennzeichnet, dass** das Fluid mindestens eines der Folgenden ist: Blut, Kontrastmittel, interstitielle Flüssigkeit, Lymphflüssigkeit, Stoffwechselflüssigkeit, Samenflüssigkeit, Fruchtwasser, Gas, Masse eines Verdauungstrakts und Flüssigkeit eines Harntrakts.

10. Einrichtung nach einem der vorstehenden Ansprüche, **dadurch gekennzeichnet, dass** die optische Strahlungsquelle (12) dazu ausgebildet ist, den optischen Impuls aus nahinfrarotem Licht wiederholt auszugeben.

11. Einrichtung nach einem der vorstehenden Ansprüche, **dadurch gekennzeichnet, dass** die optische Strahlungsquelle (12) dazu ausgebildet ist, den optischen Impuls aus nahinfrarotem Licht in einem oder mehreren Bändern, die, basierend auf mindestens einem der folgenden, ausgewählt sind, wiederholt auszugeben: optische Eigenschaften des Gewebes und optische Eigenschaften des Fluids.

12. Einrichtung nach einem der vorstehenden Ansprüche, **dadurch gekennzeichnet, dass** die Datenverarbeitungsschaltung (16) dazu ausgebildet ist, die Geschwindigkeit der Strömung als ein Verhältnis zwischen der bekannten Entfernung und einem zeitlichen Unterschied zwischen Detektionen von Photonen, die durch das mindestens eine erste Array (14A) und das mindestens eine zweite Array (14B) durchgeführt werden, zu bestimmen, wobei der zeitliche Unterschied auf einem Peak der Ähnlichkeit basiert.

13. Einrichtung nach einem der vorstehenden Ansprüche, **dadurch gekennzeichnet, dass** das Fluid Blut ist und die Datenverarbeitungsschaltung (16) dazu ausgebildet ist, einen Herzschlag basierend auf einer charakteristischen Variation der Anzahlen von Photonen als Funktion der Zeit, zu bestimmen.

14. Einrichtung nach einem der vorstehenden Ansprüche, **dadurch gekennzeichnet, dass** die Datenverarbeitungsschaltung (16) einen oder mehrere Prozessoren (500) und einen oder mehrere Speicher (502), die Computerprogrammcode einschließen, umfasst; wobei die einen oder mehreren Speicher (502) und der Computerprogrammcode dazu ausgebildet sind, zusammen mit den einen oder mehreren Prozessoren (500) zu veranlassen, dass die Einrichtung mindestens Folgendes ausführt:
Bestimmen der Geschwindigkeit der Strömung, basierend auf der Geometrie des mindestens einen ersten und zweiten Arrays (14A, 14B) und der Ähnlichkeit zwischen Anzahlen von Photonen, die durch das mindestens eine erste und zweite Array (14A, 14B) an der Vielzahl von Zeitpunkten von Ausgaben des optischen Impulses detektiert werden.

15. Optisches Verfahren zum Messen einer Fluidströmung innerhalb eines lebenden Gewebes, umfassend
Ausgeben (800) eines optischen Impulses in das Gewebe (10) durch eine optische Strahlungsquelle (12) zu aufeinanderfolgenden Zeitpunkten;
Detektieren (802) von Photonen aus einer Wechselwirkung zwischen dem optischen Impuls und der Fluidströmung an einem ersten Ort der Strömung durch mindestens ein erstes Array (14A) von Einzelphotonen-Lawinendetektoren für jeden der aufeinanderfolgenden Zeitpunkte der Ausgaben des optischen Impulses;
Detektieren (804), durch mindestens ein zweites Array (14B) von Einzelphotonen-Lawinendetektoren, für jeden der aufeinanderfolgenden Zeitpunkte der aufeinanderfolgenden Ausgaben des optischen Impulses Photonen aus Wechselwirkung zwischen dem optischen Impuls und der Fluidströmung an einem zweiten Ort der Strömung, wobei der zweite Ort in Bezug auf den ersten Ort an einem stromaufwärts oder stromabwärts gelegenen Ort liegt; und
Bestimmen (806) einer Geschwindigkeit der Strömung durch eine Datenverarbeitungsschaltung (16), basierend auf einer bekannten Geometrie des mindestens einen ersten und zweiten Arrays (14A, 14B) und einer Ähnlichkeit zwischen Anzahlen von Photonen, die durch das mindestens eine erste und zweite Array (14A, 14B) an einer Vielzahl von Zeitpunkten von Ausgaben des optischen Impulses detektiert werden.

## Revendications

1. Appareil optique pour mesurer un écoulement de fluide dans un tissu (10) vivant, dans lequel l'appareil comprend
une source de rayonnement optique (12) configurée pour émettre une impulsion optique dans le tissu (10) à des moments successifs ;
au moins un premier réseau (14A) de détecteurs à avalanche monophotonique configuré pour détecter, pour chacun des moments successifs auxquels l'impulsion optique est émise, des photons provenant d'une interaction entre l'impulsion optique et l'écoulement de fluide à un premier emplacement de l'écoulement ;
au moins un deuxième réseau (14B) de détecteurs à avalanche monophotonique configuré pour détecter, pour chacun des moments successifs auxquels l'impulsion optique est émise de façon successive, des photons provenant d'une interaction entre l'impulsion optique et l'écoulement de fluide à un deuxième emplacement de l'écoulement, le deuxième emplacement étant à un emplacement en amont ou en aval par rapport au premier emplacement ; et
un circuit de traitement de données (16) configuré pour déterminer une vitesse de l'écoulement sur la base d'une géométrie connue de l'au moins un premier réseau (14A) et de l'au moins un deuxième réseau (14B), et d'une similarité entre des nombres de photons détectés par l'au moins un premier réseau et deuxième réseau (14A, 14B) à une pluralité de moments auxquels l'impulsion optique est émise.

2. Appareil selon la revendication 1, **caractérisé en ce que** l'au moins un premier réseau (14A) et l'au moins un deuxième réseau (14B) sont mobiles par rapport au tissu (10), et simultanément au mouvement des réseaux (14A, 14B) le circuit de traitement de données (16) est configuré pour compter un nombre de photons détectés par l'au moins un premier réseau (14A) et un nombre de photons détectés par l'au moins un deuxième réseau (14B) de manière synchrone avec chacune des fois où l'impulsion optique est émise ; et
le circuit de traitement de données (16) est configuré pour former et présenter des informations sur les nombres de photons détectés par les réseaux (14A, 14B) en fonction d'un emplacement se rapportant au tissu (10) pour guider un utilisateur afin de placer les réseaux (14A, 14B) à des positions où l'écoulement est détectable pour la mesure de la vitesse.

3. Appareil selon la revendication 1, **caractérisé en ce que** le circuit de traitement de données (16) est configuré pour compter un nombre de photons détectés par l'au moins un premier réseau (14A) et l'au moins un deuxième réseau (14B) dans une ou plusieurs fenêtres temporelles (TW) dans une période de mesure (500) conjointement à chacun des moments successifs auxquels l'impulsion optique est émise, et former et présenter des informations sur un nombre de photons dans une ou plusieurs profondeurs dans le tissu (10), chacune des fenêtres temporelles (TW) dans la période de mesure (500) correspondant à une seule plage de profondeur (D*i*) de la plage totale de profondeur de mesure (TMDR) dans le tissu (10) pour guider l'utilisateur afin de mesurer la vitesse d'un écoulement dans une ou plusieurs plages de profondeur souhaitées (D*k* à D*j*).

4. Appareil selon la revendication 1, **caractérisé en ce que** le circuit de traitement de données (16) est configuré pour compter un nombre de photons détectés par l'au moins un premier réseau (14A) et l'au moins un deuxième réseau (14B) dans une ou plusieurs fenêtres temporelles (TW) dans une période de mesure (500) conjointement à chacun des moments successifs auxquels l'impulsion optique est émise, et déterminer au moins une fenêtre temporelle (TW) dans la période de mesure (500) pour mesurer la vitesse de l'écoulement à chaque plage de profondeur (D*i*) correspondant à ladite au moins une fenêtre temporelle (TW).

5. Appareil selon la revendication 1, **caractérisé en ce qu'**un circuit de traitement de données (16) est configuré pour déterminer, pour chacun des moments successifs conjointement à chacune des fois où l'impulsion optique est émise, un intervalle de temps (510) entre une émission de l'impulsion optique et une réception de photons de l'impulsion optique ayant interagi avec l'écoulement séparément pour l'au moins un premier réseau (14A) et l'au moins un deuxième réseau (14B).

6. Appareil selon la revendication 1 ou la revendication 5, **caractérisé en ce que** le circuit de traitement de données (16) est configuré pour déterminer la vitesse de l'écoulement sur la base de la similarité entre des distributions temporelles de nombres de détections de photons par l'au moins un premier réseau (14A) et, l'au moins un deuxième réseau (14B) à une pluralité de moments successifs dans une période de mesure conjointement à au moins un des moments successifs auxquels l'impulsion optique est émise.

7. Appareil selon la revendication 1, **caractérisé en ce que** l'au moins un premier réseau (14A) et l'au moins un deuxième réseau (14B) sont intégrés ensemble en tant qu'un seul réseau (14) en matériau solide.

8. Appareil selon l'une quelconque des revendications précédentes, **caractérisé en ce qu'**un circuit pilote électrique (600) de la source de rayonnement optique (10), les premier et deuxième réseaux (14A, 14B) et au moins une partie du circuit de traitement de données (16) sont fabriqués sur un matériau semi-conducteur d'une même puce.

9. Appareil selon l'une quelconque des revendications précédentes, **caractérisé en ce que** le fluide est au moins un des éléments suivants : sang, milieu de contraste, fluide interstitiel, fluide lymphatique, fluide du métabolisme, liquide séminal, liquide amniotique, gaz, masse d'un tractus alimentaire et liquide d'un tractus urinaire.

10. Appareil selon l'une quelconque des revendications précédentes, **caractérisé en ce que** la source de rayonnement optique (12) est configurée pour émettre de façon répétée l'impulsion optique de lumière proche infrarouge.

11. Appareil selon l'une quelconque des revendications précédentes, **caractérisé en ce que** la source de rayonnement optique (12) est configurée pour émettre de façon répétée l'impulsion optique de lumière proche infrarouge dans une ou plusieurs bandes qui sont choisies sur la base d'au moins un des éléments suivants : propriétés optiques du tissu et propriétés optiques du fluide.

12. Appareil selon l'une quelconque des revendications précédentes, **caractérisé en ce que** le circuit de traitement de données (16) est configuré pour déterminer la vitesse de l'écoulement en tant que rapport entre la distance connue et une différence temporelle entre des détections de photons effectuées par l'au moins un premier réseau (14A) et l'au moins un deuxième réseau (14B), la différence temporelle étant sur la base d'un pic de similarité.

13. Appareil selon l'une quelconque des revendications précédentes, **caractérisé en ce que** le fluide est du sang et le circuit de traitement de données (16) est configuré pour déterminer un battement cardiaque sur la base d'une variation caractéristique des nombres de photons en fonction du temps.

14. Appareil selon l'une quelconque des revendications précédentes, **caractérisé en ce que** le circuit de traitement de données (16) comprend un ou plusieurs processeurs (500), et une ou plusieurs mémoires (502) incluant du code de programme informatique ; les une ou plusieurs mémoires (502) et le code de programme informatique étant configurés pour, avec les un ou plusieurs processeurs (500), amener l'appareil au moins à :
déterminer la vitesse de l'écoulement sur la base de la géométrie de l'au moins un premier réseau et deuxième réseau (14A, 14B), et de la similarité entre des nombres de photons détectés par l'au moins un premier et deuxième réseau (14A, 14B) à la pluralité de moments d'émissions de l'impulsion optique.

15. Procédé optique de mesure d'un écoulement de fluide dans un tissu vivant, comprenant
l'émission (800), par une source de rayonnement optique (12), d'une impulsion optique dans le tissu (10) à des moments successifs ;
la détection (802), par au moins un premier réseau (14A) de détecteurs à avalanche monophotonique, pour chacun des moments successifs auxquels l'impulsion optique est émise, des photons provenant d'une interaction entre l'impulsion optique et l'écoulement de fluide à un premier emplacement de l'écoulement ;
la détection (804), par au moins un deuxième réseau (14B) de détecteurs à avalanche monophotonique, pour chacun des moments successifs auxquels l'impulsion optique est émise de façon successive, des photons provenant d'une interaction entre l'impulsion optique et l'écoulement de fluide à un deuxième emplacement de l'écoulement, le deuxième emplacement étant à un emplacement en amont ou en aval par rapport au premier emplacement ; et
la détermination (806), par un circuit de traitement de données (16), d'une vitesse de l'écoulement sur la base d'une géométrie connue de l'au moins un premier réseau et deuxième réseau (14A, 14B), et d'une similarité entre des nombres de photons détectés par l'au moins un premier et deuxième réseau (14A, 14B) à une pluralité de moments d'émissions de l'impulsion optique.
